# FASCICULE DE BREVET EUROPEEN

(11) **EP 1 315 525 B1**
(45) Date de publication et mention de la délivrance du brevet: **25.08.2004**
(21) Numéro de dépôt: 01913962.5
(22) Date de dépôt: 08.03.2001
(51) Int. Cl.: A61L 2/12, A61L 2/02, B27K 5/00, B08B 9/08

(54) **DESINFECTION D'ELEMENTS EN BOIS EN CONTACT AVEC DES DENREES ALIMENTAIRES**
DESINFEKTION VON HOLZELEMENTEN, DIE IN KONTAKT MIT NAHRUNGSMITTELN STEHEN
DISINFECTING WOODEN ELEMENTS IN CONTACT WITH FOODSTUFF

(30) Priorité: 10.03.2000 FR 0003079
(43) Date de publication de la demande: 04.06.2003
(73) Titulaire: Thales International, 33610 Canejan (FR)
(72) Inventeur: BLONDEAU, Eric, F-64200 Biarritz (FR); BERTHELOT, Sophie, F-33130 Begles (FR)
(74) Mandataire: Dronne, Guy
(86) Numéro de dépôt international: PCT/FR2001/000693
(87) Numéro de publication internationale: WO 2001/068153

(56) Documents cités:
- DE-C- 19 802 297
- GB-A- 1 126 233

## Description

La présente invention concerne un procédé et une installation de désinfection d'un fût en bois susceptible d'avoir été contaminé par des micro-organismes lors de son utilisation.

Un domaine d'application envisagé est notamment mais non exclusivement celui de la stérilisation des fûts destinés au vieillissement du vin ou des fûts susceptibles de contenir des denrées alimentaires.

Généralement le vin vieillit en fût pendant une période durant laquelle les constituants du vin diffusent et réagissent avec le bois de la paroi interne et des couches plus profondes du fût. Ensuite, le vin est soutiré et il reste éventuellement des dépôts sur la paroi interne du fût. Pour être utilisé à nouveau dans une application similaire ou identique, le fût doit être reconditionné pour éliminer les dépôts éventuels puis désinfecté.

Le reconditionnement consiste à nettoyer la paroi interne du fût et à la désinfecter ensuite pour obtenir un état sanitaire satisfaisant et une conservation optimale des fûts.

Habituellement, l'intérieur des fûts est désinfecté ou stérilisé par l'action de vapeur d'eau, qui peut être injectée sous pression et maintenue à l'intérieur pendant un temps déterminé. Ainsi, l'énergie thermique détruit les micro-organisme situés dans le fût. Cependant, la destruction intervient avec un certain retard compte tenu de l'inertie thermique des parois du fût et grève le rendement du procédé. Afin d'accélérer la vitesse de désinfection, il est possible d'augmenter la pression de vapeur d'eau dans le fût, mais cela fait courir un risque d'explosion non négligeable et/ou accroît le lessivage des parois internes du fût.

Un autre inconvénient de la stérilisation par la vapeur est qu'elle contribue à faire gonfler le bois qui absorbe de l'eau et le rend plus malléable.

Un objet de la présente invention est de proposer un procédé de désinfection du bois susceptible d'avoir été contaminé par les micro-organismes en évitant les inconvénients liés à l'utilisation de la vapeur d'eau.

Pour atteindre ce but, selon l'invention, le procédé de désinfection d'un fût en bois susceptible d'avoir été contaminé par des micro-organismes, se caractérise en ce que l'on soumet ledit fût à l'action d'un champ électromagnétique dont la fréquence est comprise entre 1 MHz et 300 GHz, pour détruire au moins une partie de la population des micro-organismes qu'il comporte.

L'action du champ électromagnétique dans la gamme de fréquence allant de 1MHz à 300 GHz produit une oscillation des molécules polaires ou des ions qui, par friction, provoque un échauffement de la matière. Le transfert thermique par conduction joue un rôle secondaire au sein de la masse, contrairement aux autres techniques de chauffage, mais il équilibre la température localement.

Les fréquences utilisées présentent l'avantage d'avoir un rayonnement dont la pénétration est de quelques centimètres alors que la pénétration du rayonnement infrarouge, dont la longueur d'onde est comprise entre 10⁻³ m et 10⁻⁶ m et qui est couramment utilisé pour chauffer la matière, est de quelques millimètres. En conséquence, il est possible de provoquer un échauffement de la surface interne de la paroi lorsque son épaisseur est de quelques centimètres par friction des molécules polaires ou des clusters, sensiblement en tout point et au même instant. De la sorte, les micro-organismes fixés sur la surface interne de la paroi du contenant sont détruits par l'action du rayonnement, qui permet d'augmenter la température de l'environnement des micro-organismes.

Préférentiellement, le fût en bois est destiné à contenir des alcools ou des denrées alimentaires. Ces fûts sont constitués par des éléments longitudinaux en bois accolés hermétiquement les uns aux autres pour former un récipient de symétrie cylindrique et retenus par des cercles généralement métalliques, les deux extrémités du récipient étant obturés par des fonts plats. Alors que la présence des cercles métalliques interdisait que l'on puisse envisager de traiter les fûts par un rayonnement électromagnétique approprié, qui aurait provoqué des arcs électriques préjudiciables au traitement et notamment des échauffements locaux susceptibles de détériorer la paroi, il s'avère que la surface interne de la paroi du fût s'échauffe sans que des arcs électriques n'apparaissent au niveau des cercles métalliques. Ainsi le traitement des fûts en bois par un rayonnement électromagnétique approprié conduit à une stérilisation optimale de sa paroi interne d'une part, qui est en contact direct avec le liquide qu'il est susceptible de contenir, et de l'ensemble du fût d'autre part. Bien qu'un fût soit un contenant fermé, l'épaisseur de sa paroi étant de quelques centimètres, le rayonnement produit un effet sur sa surface interne.

Outre l'action directe sur les micro-organismes, le rayonnement agit sur leur environnement et en particulier sur l'eau qui est inévitablement présente en plus ou moins grande quantité sur ou dans la paroi et qui est la molécule polaire par excellence. La friction des molécules d'eau produit une élévation de la température contribuant également à la dégradation des micro-organismes.

Selon un mode préféré de mise en oeuvre, la fréquence du champ électromagnétique est comprise entre 865 MHz et 5850 MHz, ce qui correspond à des micro-ondes.

Selon une caractéristique particulière, le fût en bois est mobile par rapport à une source électromagnétique pour que l'action dudit champ sur ledit fût soit uniforme. Ainsi, les points chauds, distants d'une demi-longueur d'onde qui surviennent lorsque l'on irradie un objet s'atténuent par conduction puisque le contenant est mobile par rapport à la source.

Selon une autre caractéristique particulière, la source électromagnétique est mobile par rapport audit fût en bois de façon à pouvoir traiter les contenants en bois qui sont fixes ou trop volumineux pour être transportés, c'est par exemple le cas des foudres et des tonneaux, et à résoudre le problème de l'homogénéisation de la température au sein du contenant.

Selon une troisième caractéristique particulière, on applique un champ électromagnétique de 2450 MHz plus ou moins 25 MHz dont la puissance restituée est supérieure à 5 kW durant un temps au moins égal à 300 s pour un contenant standard. Ainsi, la quantité d'énergie appliquée sur le contenant en bois est suffisante pour détruire au moins une partie de la population des micro-organismes qu'il comporte.

Un autre objet de la présente invention est de fournir une installation de stérilisation ou de désinfection d'au moins un fût en bois susceptible d'avoir été contaminé par des micro-organismes lors de son utilisation. L'installation comprend des moyens de traitement pour soumettre ledit contenant à l'action d'un champ électromagnétique dont la fréquence est comprise entre 1 MHz et 300 GHz, de façon à détruire au moins une partie de la population des micro-organismes qu'il comporte.

De façon avantageuse, les moyens de traitement comprennent au moins un générateur d'onde électromagnétique prolongé par un applicateur, qui y est connecté, apte à faire interagir lesdites ondes sur ledit fût en bois. L'applicateur est constitué d'une antenne à fentes formant guide d'onde pour faire interagir les ondes précisément sur le fût en bois.

Préférentiellement, l'installation comprend une pluralité de générateurs d'onde électromagnétique prolongés par des guides d'onde qui entourent au moins partiellement ledit fût en bois. Cette disposition permet de traiter le fût en bois sous plusieurs angles simultanément et conduit à une application sensiblement uniforme des surfaces du fût.

Selon une caractéristique particulière de l'invention, l'installation comprend des générateurs d'onde électromagnétique émettant des ondes dont la fréquence est comprise entre 1 MHz et 300 GHz et ayant une puissance restituée au moins égale à 1 kW.

Ces caractéristiques permettent la destruction des micro-organismes puisque l'énergie thermique consécutive à l'action des ondes dépasse une valeur au-delà de laquelle ils ne peuvent survivre.

Selon une autre caractéristique particulière on prévoit des moyens support situés à proximité des guides d'onde aptes à supporter ledit fût en bois.

De façon avantageuse les moyens support sont mobiles pour déplacer ledit fût en bois par rapport aux guides d'onde de manière à homogénéiser l'action des ondes sur ledit fût et à traiter uniformément le fut.

Selon un mode particulier de mise en oeuvre les moyens de traitement sont mobiles par rapport audit fût pour un traitement uniforme ou pour pouvoir être transportés sur le lieu d'utilisation pour des fût fixes en bois.

D'autres particularités et avantages de l'invention ressortiront à la lecture de la description faite ci-après de modes de réalisation particuliers de l'invention, donnés à titre indicatif mais non limitatif, en référence aux dessins annexés sur lesquels :
- la Figure 1 est une vue en perspective montrant une installation selon un mode particulier de mise en oeuvre dans lequel trois générateurs munis de trois guides d'onde parallèles sont installés,
- la Figure 2, est une vue de dessous montrant une installation selon un autre mode particulier de réalisation dans lequel trois guides d'onde sont montés à 120° les uns des autres dans un même plan,
- la Figure 3 est une vue de dessus montrant l'installation selon le mode de réalisation de la Figure 2,
- la Figure 4 est une vue de face montrant une installation selon un troisième mode de réalisation dans lequel un fût est placé entre deux guides d'onde verticaux, et,
- la Figure 5 est une vue en perspective montrant une installation selon un quatrième mode de réalisation dans lequel un fût est placé entre trois paires de guides d'onde prolongeant chacun un générateur.

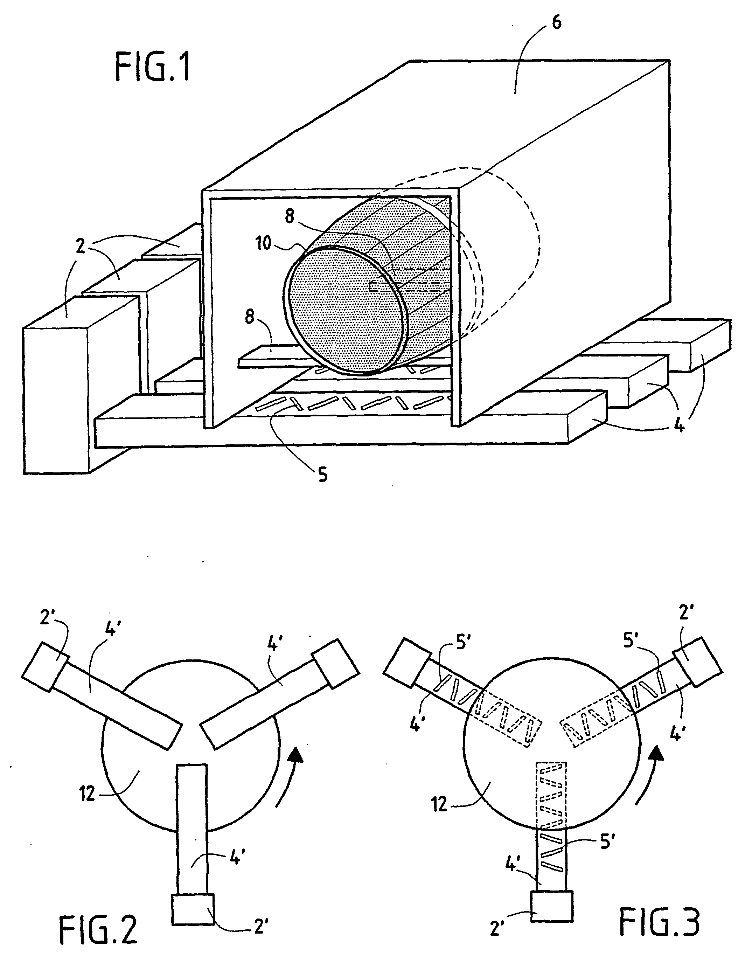

En se référant tout d'abord à la Figure 1 on va décrire une installation pour la mise en oeuvre du procédé conformément à l'invention.

L'installation comprend trois blocs générateurs d'onde 2 prolongés par trois guides d'onde 4. Les blocs générateurs d'onde 2 sont constitués d'un magnétron apte à produire des miro-ondes dont la fréquence est de 2450 MHz à plus ou moins 25 MHz. Cette fréquence n'est pas limitative et toute autre fréquence pourrait être envisagée à condition de respecter les normes en vigueurs, ou de concevoir une installation parfaitement isolée.

Le bloc 2 comprend également une protection thermique pour le magnétron de manière à éviter le risque d'une détérioration par une montée en température trop importante.

La puissance restituée du magnétron, apte à produire des ondes dont la fréquence est de 2450 MHz à plus ou moins 25 MHz, est de 1,2 kW mais elle est nullement limitative et peut être adaptée en fonction du traitement envisagé.

Les ondes électromagnétiques générées par le magnétron sont dirigées dans une cavité appelée guide d'onde 4 dans laquelle elles se propagent. Les cavités représentées sur la Figure 1 sont munies de fentes 5 alternées que les ondes traversent pour se propager à l'extérieur. Ces fentes 5 ont une forme et une disposition particulières pour éviter les couplages et optimiser l'émission de l'onde de manière à éviter son retour vers le générateur. En particulier, elles sont sensiblement et consécutivement à 90°les unes des autres.

Les trois guides d'ondes 4 sont disposés parallèlement et sont contigus de manière à former une surface d'émission plane.

L'installation comporte un caisson 6 constituant une barrière électromagnétique qui est fixée sur cette surface plane et qui peut également être munie d'un dispositif d'aspiration, non représenté, apte à aspirer les gaz et les substances volatiles que le traitement micro-ondes produirait.

Le caisson 6 présente des supports fixes 8 sur lesquels un fût en bois 10 à traiter est appuyé. Le type de fût en bois susceptible d'être soumis à l'action des micro-ondes à des fins de stérilisation est extrêmement large, mais généralement il s'agit de fût utilisés dans les industries agro-alimentaires dans lesquelles la prolifération de germes ou de micro-organismes doit être absolument limitée.

Par ailleurs, les micro-organismes sont susceptibles de se développer non seulement sur les surfaces des parois des fûts en bois mais également dans les couches profondes, ce qui présente une difficulté supplémentaire quant au procédé de désinfection par rapport à des fûts en matières plastiques ou métalliques pour lesquels la prolifération des micro-organismes est essentiellement superficielle.

Lorsque les micro-organismes prolifèrent uniquement en surface, le traitement est plus aisé et peut se faire en augmentant la température superficielle du contenant par de la vapeur d'eau ou par une source thermique quelconque.

En revanche, pour le bois, où les micro-organismes sont susceptibles de se développer dans toute l'épaisseur, la destruction thermique n'est réalisée que par l'augmentation de température de tout le fût. Or, dans le cas d'un chauffage par radiation infrarouge ou par la vapeur d'eau dont la pénétration dans les couches superficielles est faible ou nulle, la température n'augmente au coeur du fût que par conduction, ce qui nécessite un certain temps.

Le rayonnement micro-ondes à 2450 MHz à plus ou moins 25 MHz présente l'avantage d'être beaucoup plus pénétrant que le rayonnement infra-rouge et pénètre dans une épaisseur de quelques centimètres. Ainsi, pour un fût en bois dont l'épaisseur est du même ordre de grandeur, toute population de micro-organisme, quelle que soit sa profondeur, est substantiellement détruite par le chauffage de son environnement.

Cette destruction procède de l'action indirecte de l'échauffement des composés polaires entourant ces micro-organismes. Les fûts en bois situés dans les conditions atmosphériques normales présentent un taux d'humidité substantiel, et c'est principalement l'eau qui s'échauffe également dans le bois et qui concourt à la destruction thermique des micro-organismes.

L'échauffement interne du fût en bois peut produire de la vapeur qui induit une surpression interne accroissant l'effet thermique et donc l'action sur les micro-organismes.

Sur la Figure 1, le fût en bois 10 est destiné à contenir du vin ou tout autre liquide alimentaire. Après qu'il a été posé sur les supports 8, le fût en bois 10 est soumis à l'action des micro-ondes par la mise en fonctionnement des magnétrons qui produisent un rayonnement se propageant dans les guides d'onde 4 et qui traverse les fentes 5 pour agir sous ledit contenant 10. Les ondes traversent la paroi du fût de sorte qu'elles l'échauffent dans toute sa masse, et en particulier sa surface interne, de sorte que la totalité des micro-organismes qu'elle comporte est susceptible d'être touchée et donc détruite.

En se référant maintenant aux Figures 2 et Figure 3, on décrira un mode particulier de mise en oeuvre de l'installation qui tient compte des problèmes de discontinuité d'échauffement que peut procurer le traitement micro-ondes.

En effet, dans l'installation précédente, le contenant en bois 10 est statique par rapport à la source et les fûts de volume sont inégalement soumis au rayonnement ce qui produit des zones de températures différentes.

Pour remédier à cet inconvénient, un plateau 12 transparent aux micro-ondes muni d'un dispositif d'entraînement en rotation et/ou en translation, est disposé au-dessus des guides d'onde 4' au regard des fentes 5'. Trois guides d'onde 4' sont disposés en étoile dans un même plan faisant un angle de 120° les uns des autres. Les blocs générateurs ne sont pas représentés mais ils se fixent à l'extrémité des trois guides d'onde 4' formant étoile.

Le fût en bois à traiter est disposé sur la surface 14 du plateau tournant 12 qui est actionné en rotation durant le fonctionnement des générateurs d'onde. Les ondes qui traversent les fentes 5' traversent également le plateau tournant 12, qui est généralement en Teflon® et transparent aux micro-ondes, pour agir sur le fût en bois, non représenté, posé à la surface 14 du plateau tournant 12.

Les ondes se propagent selon des directions constantes, et la rotation du contenant en bois permet de faire varier l'incidence des ondes sur tous ses contenants de volume, et donc d'homogénéiser l'action du champ électromagnétique sur la totalité du contenant en bois.

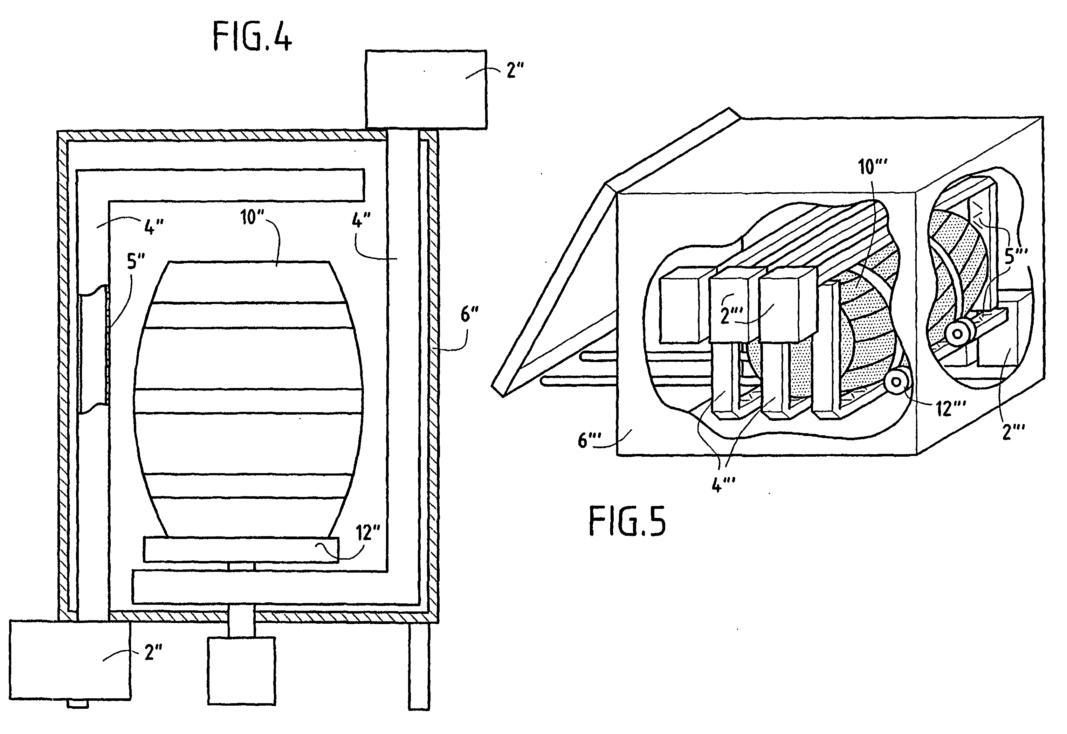

Selon un autre mode particulier de réalisation, pour lequel on se référera à la Figure 4, on décrira une installation destinée à la stérilisation des fûts en bois pour le stockage du vin ou tout autre produit alimentaire.

L'installation comprend une enceinte métallique 6", dans laquelle deux guides d'onde 4 sont disposés verticalement contre deux parois opposées avec les fentes 5" en regard les unes des autres et à l'extrémité supérieure desquels sont fixés des blocs 2" générateurs d'ondes. Un plateau tournant 12" est relié à la base de l'enceinte 6" par l'intermédiaire d'un dispositif apte à l'actionner en rotation.

Un fût 10" est disposé verticalement sur le plateau tournant 12" entre les deux guides d'onde 4" et il est actionné en rotation durant le fonctionnement des générateurs 2". En conséquence, tout comme dans le mode de réalisation précédent, l'incidence des ondes sur tous les contenants de volume du fût 10" varie avec son mouvement et permet l'homogénéisation thermique.

Les fûts utilisés pour le vieillissement du vin comportent certains types de micro-organismes dont il faut détruire la majorité des populations. Ces micro-organismes sont des bactéries acétiques, par exemple des *Acetobacter,* des levures, par exemple des *Brettanomyces,* des bactéries lactiques, par exemple des *Pediococcus* ou des moisissures diverses du type *Penicillium,* ou encore tout autre organisme qui persiste après le soutirage du vin et qui est susceptible de tapisser la surface interne du fût 10" et les couches plus profondes.

Les micro-ondes conviennent parfaitement à la destruction des micro-organismes se développant à l'intérieur du fût 10" puisque le rayonnement est suffisamment pénétrant pour agir sur sa surface interne. Par ailleurs, tout le volume de la paroi du fût 10" est soumis au rayonnement, par conséquent tout agent contaminant présent à l'intérieur de la paroi peut être atteint.

Selon un troisième mode particulier de mise en oeuvre de l'invention, illustré sur la Figure 5, l'installation comprend six blocs générateurs d'ondes 2"'chacun étant muni d'un guide d'onde 4"' formant un "L". Les guides d'onde 4"' et leurs blocs générateurs sont associés par paire de manière à former un quadrilataire, les trois paires étant juxtaposées afin de constituer un parallélépipède apte à entourer un fût 10"' horizontal présentant une contenance de 225 litres dans le cas standard.

Les guides d'onde comportent des fentes 5"' dirigées vers le fût 10"' sur les faces internes des deux branches des guides d'onde formant un "L".

L'installation présente également deux paires de galets 12"', sur lesquels le fût 10"' est posé, ils sont actionnés en rotation par une motorisation de manière à entraîner le fût 10"' autour de son axe principal durant la mise en fonctionnement des générateurs.

L'ensemble est entouré d'une enceinte 6"' de façon à contenir les ondes électromagnétiques générées par les magnétrons.

Afin d'illustrer l'invention selon le mode de réalisation précédemment décrit un exemple de conditions opératoires est donné à titre d'illustration.

Les fûts à traiter dont la contenance est comprise entre 200 et 600 litres présentent une hygrométrie comprise entre 10 et 50% ; ils ont été utilisé pour le vieillissement d'un alcool et ils sont traités pour être réutilisés.

Au préalable ils sont nettoyés avec de l'eau contenant éventuellement des produits détergents puis ils sont introduits un par un dans l'installation décrite ci-dessus.

Les générateurs ont une puissance de 1,2 kW chacun et la fréquence dans laquelle ils travaillent est de 2450 MHz plus ou moins 25 MHz. Le temps de traitement est de 900 secondes et la température de la surface interne du fût est comprise entre 65 et 95°C tandis que la température interne de la paroi du fût est comprise entre 50 et 90°C.

Dans ces conditions opératoires les populations de micro-organismes initialement contenues dans le fût ont été détruites et permettent d'obtenir un fût désinfecté apte à être utilisé à nouveau pour le stockage du vin.

Pour tous les modes de réalisation décrits ci-dessus, on prévoit de pouvoir adapter à l'installation des moyens thermiques additionnels aptes à fournir une énergie thermique supplémentaire audit contenant en bois. Cette énergie est susceptible d'être fournie par de la vapeur d'eau ou tout autre moyen. Cette adjonction est d'autant plus intéressante pour apporter un complément de traitement sur toute partie en bois située sous les cerdes métalliques.

Par ailleurs, le générateur d'onde est susceptible d'émettre des ondes par impulsion ou en continu.

## Revendications

1. Procédé de désinfection de fûts en bois susceptibles de former un contenant fermé et d'avoir été contaminés par des micro-organismes, **caractérisé en ce que** l'on soumet ledit fût en bois (10, 10", 10"') à l'action d'un champ électromagnétique dont la fréquence est comprise entre 1 MHz et 300 GHz, de façon à atteindre la surface interne de la paroi dudit fût en bois pour détruire au moins une partie de la population des micro-organismes que ledit fût comporte.

2. Procédé de désinfection selon la revendication 1, **caractérisé en ce que** ledit fût en bois (10", 10"') est constitué par des éléments longitudinaux en bois accolés hermétiquement les uns aux autres et retenus par des cercles métalliques.

3. Procédé de désinfection selon l'une quelconque des revendications 1 à 2, **caractérisé en ce que** la fréquence du champ électromagnétique est comprise entre 865 MHz et 5850 MHz.

4. Procédé de désinfection selon l'une quelconque des revendications 1 à 3, **caractérisé en ce que** ledit fût en bois (10, 10", 10"') est mobile par rapport à une source électromagnétique pour que l'action dudit champ sur ledit fût soit uniforme.

5. Procédé de désinfection selon l'une quelconque des revendications 1 à 4, **caractérisé en ce que** la source électromagnétique est mobile par rapport audit fût en bois.

6. Procédé de désinfection selon l'une quelconque des revendications 1 à 5, **caractérisé en ce que** l'on applique un champ électromagnétique de 2450 MHz plus ou moins 25 MHz dont la puissance restituée est supérieure à 5 kW durant un temps au moins égal à 300 s pour un fût en bois standard.

7. Installation de désinfection d'au moins un fût en bois (10, 10", 10"') susceptible d'avoir été contaminé par des micro-organismes, **caractérisée en ce qu'**elle comprend des moyens de traitement pour soumettre ledit fût à l'action d'un champ électromagnétique dont la fréquence est comprise entre 1 MHz et 300 GHz, de façon à détruire au moins une partie de la population des micro-organismes qu'il comporte.

8. Installation de désinfection selon la revendication 7, **caractérisée en ce que** les moyens de traitement comprennent au moins un générateur (2) d'onde électromagnétique prolongé par un applicateur (4) apte à faire interagir lesdites ondes sur ledit fût en bois.

9. Installation de désinfection selon la revendication 8, **caractérisée en ce que** ledit applicateur est constitué d'une antenne à fentes.

10. Installation de désinfection selon la revendication 8 ou 9, **caractérisée en ce qu'**elle comprend une pluralité de générateurs (2, 2', 2", 2"') d'onde électromagnétique prolongés par des applicateurs (4) qui entourent au moins partiellement ledit fût en bois.

11. Installation de désinfection selon l'une quelconque des revendications 8 à 10, **caractérisée en ce qu'**elle comprend des générateurs (2, 2', 2", 2"') d'onde électromagnétique émettant des ondes dont la fréquence est comprise entre 865 MHz et 5850 MHz.

12. Installation de désinfection selon l'une quelconque des revendications 8 à 11, **caractérisée en ce qu'**elle comprend en outre des moyens support (8) situés à proximité des applicateurs (4) et aptes à supporter ledit fût en bois.

13. Installation de désinfection selon la revendication 12, **caractérisée en ce qu'**elle comprend en outre des moyens support (12, 12", 12"') mobiles pour déplacer ledit fût en bois (10, 10", 10"') par rapport aux applicateurs (4) de manière à homogénéiser l'action des ondes sur ledit fût.

14. Installation de désinfection selon la revendication 7, **caractérisée en ce que** les moyens de traitement sont mobiles par rapport audit fût en bois.

15. Installation de désinfection selon l'une quelconque des revendication 7 à 14, **caractérisée en ce qu'**elle comprend en outre des moyens thermiques additionnels aptes à fournir une énergie thermique supplémentaire audit fût en bois.

## Patentansprüche

1. Verfahren zur Desinfektion von Behältern aus Holz, die ein geschlossenes Behältnis bilden und durch Mikroorganismen kontaminiert worden sein können, **dadurch gekennzeichnet, dass** der Holzbehälter (10, 10", 10"') der Wirkung eines elektromagnetischen Feldes ausgesetzt wird, dessen Frequenz zwischen 1 MHz und 300 GHz liegt, um die Innenfläche der Wand des Holzbehälters zu erreichen, um zumindest einen Teil der Population der Mikroorganismen, die der Behälter umfasst, zu zerstören.

2. Verfahren zur Desinfektion nach Anspruch 1, **dadurch gekennzeichnet, dass** der Holzbehälter (10", 10"') von Längselementen aus Holz gebildet ist, die hermetisch dicht aneinander gebaut sind und durch Metallreifen gehalten werden.

3. Verfahren zur Desinfektion nach einem der Ansprüche 1 bis 2, **dadurch gekennzeichnet, dass** die Frequenz des elektromagnetischen Feldes zwischen 865 MHz und 5850 MHz liegt.

4. Verfahren zur Desinfektion nach einem der Ansprüche 1 bis 3, **dadurch gekennzeichnet, dass** der Holzbehälter (10, 10", 10''') in Bezug auf eine elektromagnetische Quelle beweglich ist, damit die Wirkung des Feldes auf den Behälter gleichmäßig ist.

5. Verfahren zur Desinfektion nach einem der Ansprüche 1 bis 4, **dadurch gekennzeichnet, dass** die elektromagnetische Quelle in Bezug auf den Holzbehälter beweglich ist.

6. Verfahren zur Desinfektion nach einem der Ansprüche 1 bis 5, **dadurch gekennzeichnet, dass** ein elektromagnetisches Feld von 2450 MHz plus/minus 25 MHz angelegt wird, dessen erstattete Leistung größer als 5 kW während einer Zeit mindestens gleich 300 s für einen Standard-Holzbehälter ist.

7. Anlage zur Desinfektion von mindestens einem Holzbehälter (10, 10", 10"'), der mit Mikroorganismen kontaminiert worden sein könnte, **dadurch gekennzeichnet, dass** sie Behandlungsmittel umfasst, um den Behälter der Wirkung eines elektromagnetischen Feldes zu unterziehen, dessen Frequenz zwischen 1 MHz und 300 GHz liegt, um zumindest einen Teil der Population der Mikroorganismen, die er umfasst, zu zerstören.

8. Anlage zur Desinfektion nach Anspruch 7, **dadurch gekennzeichnet, dass** die Behandlungsmittel mindestens einen Generator (2) einer elektromagnetischen Welle, verlängert durch einen Applikator (4), der die Interaktion der Wellen auf dem Holzbehälter bewirken kann, umfassen.

9. Anlage zur Desinfektion nach Anspruch 8, **dadurch gekennzeichnet, dass** der Applikator von einer Schlitzantenne gebildet ist.

10. Anlage zur Desinfektion nach Anspruch 8 oder 9, **dadurch gekennzeichnet, dass** sie eine Vielzahl von Generatoren (2, 2', 2", 2"') einer elektromagnetischen Welle umfasst, die durch Applikatoren (4) verlängert sind, die zumindest zum Teil den Holzbehälter umgeben.

11. Anlage zur Desinfektion nach einem der Ansprüche 8 bis 10, **dadurch gekennzeichnet, dass** sie Generatoren (2, 2', 2", 2"') einer elektromagnetischen Welle umfasst, die Wellen aussenden, deren Frequenz zwischen 865 MHz und 5850 MHz liegt.

12. Anlage zur Desinfektion nach einem der Ansprüche 8 bis 11, **dadurch gekennzeichnet, dass** sie ferner Abstützungsmittel (8) umfasst, die sich in der Nähe der Applikatoren (4) befinden und den Holzbehälter tragen können.

13. Anlage zur Desinfektion nach Anspruch 12, **dadurch gekennzeichnet, dass** sie ferner bewegliche Abstützungsmittel (12, 12", 12''') umfasst, um den Holzbehälter (10, 10", 10''') in Bezug auf die Applikatoren (4) zu verschieben, um die Wirkung der Wellen auf den Behälter homogen zu gestalten.

14. Anlage zur Desinfektion nach Anspruch 7, **dadurch gekennzeichnet, dass** die Behandlungsmittel in Bezug auf den Holzbehälter beweglich sind.

15. Anlage zur Desinfektion nach einem der Ansprüche 7 bis 14, **dadurch gekennzeichnet, dass** sie ferner zusätzliche thermische Mittel umfasst, um eine zusätzliche Wärmeenergie zu dem Holzbehälter zu liefern.

## Claims

1. A method for disinfecting wooden casks likely to form a closed container and to have been contaminated by microorganisms, **characterised in that** said wooden cask (10, 10", 10"') is subjected to the action of an electromagnetic field, the frequency of which is between 1 MHz and 300 GHz, so as to reach the inner surface of the wall of said wooden cask in order to destroy at least part of the population of microorganisms contained in said cask.

2. The disinfection method according to claim 1, **characterised in that** said wooden cask (10", 10''') consists of longitudinal wooden elements hermetically fastened to each other and retained by metal hoops.

3. The disinfection method according to claim 1 or 2, **characterised in that** the frequency of the electromagnetic field is between 865 MHz and 5850 MHz.

4. The disinfection method according to any one of claims 1 to 3, **characterised in that** said wooden cask (10, 10", 10"') can be moved with respect to an electromagnetic source so that the action of said field on said cask is uniform.

5. The disinfection method according to any one of claims 1 to 4, **characterised in that** the electromagnetic source can be moved with respect to said wooden cask.

6. The disinfection method according to any one of claims 1 to 5, **characterised in that** an electromagnetic field of 2450 MHz plus or minus 25 MHz, the power output of which is greater than 5 kW, is applied for a time at least equal to 300 s for a standard cask.

7. A plant for disinfecting at least one wooden cask (10, 10", 10"') likely to have been contaminated by microorganisms, **characterised in that** it comprises treatment means in order to subject said cask to the action of an electromagnetic field, the frequency of which is between 1 MHz and 300 GHz, so as to destroy at least part of the population of microorganisms contained in said cask.

8. The disinfection plant according to in claim 7, **characterised in that** the treatment means comprise at least one electromagnetic wave generator (2) extended by an applicator (4) capable of making said waves interact with said wooden cask.

9. The disinfection plant according to claim 8, **characterised in that** said applicator consists of a slot antenna.

10. The disinfection plant according to claim 8 or 9, **characterised in that** it comprises a plurality of electromagnetic wave generators (2, 2', 2", 2"') extended by applicators (4) which at least partially surround said wooden cask.

11. The disinfection plant according to any one of claims 8 to 10, **characterised in that** it comprises electromagnetic wave generators (2, 2', 2", 2"') emitting waves, the frequency of which is between 865 MHz and 5850 MHz.

12. The disinfection plant according to any one of claims 8 to 11, **characterised in that** it further comprises support means (8) located close to the applicators (4) and capable of supporting said wooden cask.

13. The disinfection plant according to claim 12, **characterised in that** it further comprises support means (12, 12", 12"') which can be moved in order to displace said wooden cask (10, 10", 10"') with respect to the applicators (4) so as to homogenize the action of the waves on said cask.

14. The disinfection plant according to claim 7, **characterised in that** the treatment means can be moved with respect to said wooden cask.

15. The disinfection plant according to any one of claims 7 to 14, **characterised in that** it further comprises additional heating means capable of supplying extra heat energy to said wooden cask.
